# EUROPEAN PATENT SPECIFICATION

(11) **EP 2 396 015 B1**
(45) Date of publication and mention of the grant of the patent: **17.04.2013**
(21) Application number: 10704689.8
(22) Date of filing: 10.02.2010
(51) Int. Cl.: A61K 35/74, A61P 31/04

(54) **COMPOSITIONS COMPRISING REDUCED GENOME BACTERIA FOR USE IN TREATMENT OF SEPSIS**
ZUSAMMENSETZUNGEN MIT REDUZIERTEN GENOM-BAKTERIEN ZUR BEHANDLUNG VON SEPSIS
COMPOSITIONS COMPORTANT DES BACTÉRIES DE GÉNOME RÉDUIT POUR UTILISATION DANS LE TRAITEMENT DE LA SEPTICÉMIE

(30) Priority: 12.02.2009 US 152125 P
(43) Date of publication of application: 21.12.2011
(73) Proprietor: Scarab Genomics LLC, Madison, WI 53713 (US)
(72) Inventor: BLATTNER, Frederick, R., Madison WI 53711 (US); BURLAND, Valerie, Cross Plains WI 53528 (US); CAMPBELL, John, Walter, Chicago IL 60605 (US); LANDRY, Charles, Fitchburg WI 53711 (US)
(74) Representative: Green, Katherine
(86) International application number: PCT/US2010/023774
(87) International publication number: WO 2010/093708

## Description

### Cross-Reference to Related Applications

This application claims the benefit of U.S. Provisional Application No. 61/152,125, filed February 12, 2009.

### Field of the Invention

The present invention relates to materials and methods for the prevention of sepsis. The present invention also relates to materials and methods for the prevention of at least one symptom of sepsis. More specifically, the present invention relates to a method for preventing sepsis comprising introducing into a mammal, reduced genome bacteria lacking non-essential elements.

### Background of the Invention

Sepsis, commonly known as blood poisoning, is a serious medical condition and a major cause of morbidity and mortality in humans and other mammals. Sepsis continues to be a leading cause of death among hospitalized patients (Martin G.S. et al., N. Engl. J. Med., 348(16):1546-1554 (2003)). In the United States, sepsis is the second leading cause of death in non-coronary Internal Care Unit (ICU) patients, and the tenth most common cause of death overall according to the Centers for Disease Control and Prevention. Sepsis is a major cause of death in ICUs worldwide, with mortality rates ranging from 20% to up to 60% depending on the stage of the disease.

Sepsis is characterized by acute systemic inflammation in response to pathogenic microbes (or their toxins) in the blood with initial symptoms typically including chills, sweating, and intermittent fever, followed by persistent fever, hypotension leading to shock, neutropenia, leukopenia, disseminated intravascular coagulation, adult respiratory distress syndrome and multiple organ failure. Most symptoms of sepsis are believed to result from the host's immune response to the infection. The host response has been termed systemic inflammatory response syndrome (SIRS) and is characterized by hemodynamic compromise and resultant metabolic derangement.

Among the sepsis-inducing toxins that have been found associated with pathogenic bacteria are the endotoxins or lipopolysacchrides (LPS) unique to gram-negative bacteria such as *Escherichia coli (E. coli),* the predominant pathogen in most cases of sepsis, *Klebsiella pneumoniae* and *Pseudomonas aeruginosa.* LPS molecules are essential outer membrane glycolipids found on the cellular surface of all gram-negative bacteria. Host recognition of the lipid A region of LPS initiates many of the pathophysiologic changes of sepsis. LPS is believed to be a primary cause of death in humans during gram-negative sepsis. In plasma, LPS is released from the cell wall of bacteria and complexes with a variety of circulating lipids and proteins. Free or complexed LPS activates inflammation by acting through toll-like receptors that are present on numerous host cell types important in the immune response (including dendritic cells, intestinal epithelium, macrophages and monocytes). Molecules from bacteria (primarily endotoxin/LPS) and viruses that activate these receptors are called pathogen-associated molecular profiles (PAMPS). Upon binding to the toll-like receptors, PAMPS induce the expression of the transcription factor NF-κB which in turn causes the release of a cascade of proinflammatory cytokines such as tumor necrosis factor-alpha, interleukin 1 and interleukin 6. These factors are early players in inflammation and, if correctly regulated, follow a pattern of expression that results in localized inflammation and healing. Over-activation of this system (e.g. by PAMPS) however, results in systemic release of these cytokines, leading to excessive inflammatory response in both animals and humans during bacteremia (i.e., bacteria in the blood), injuring cells and altering blood flow, especially in the capillaries. These effects of LPS exposure lead to the accumulation of inflammatory cells in the lungs and consequent production of oxygen radicals which damage the pulmonary endothelium and initiate the acute respiratory distress syndrome often leading to death.

Damage-associated molecular profiles (DAMPS) also induce this system by activating the same receptor complement with slight modification. Unlike PAMPS, which are released from bacterial or viral pathogens (or endotoxin/LPS), DAMPS are released following cellular damage or disease. Like PAMPS, DAMPS can induce systemic inflammation, which is also a leading cause of morbidity in trauma patients.

Infection of the blood with pathogenic microbes is often acquired in a hospital setting and can result from surgical procedures, immunosuppressive cancer or transplantation therapies, immune deficiency diseases and exposure via intravenous catheters. Also at risk for developing sepsis are trauma and burn victims.

Current treatment regimens for subjects with sepsis include broad spectrum antibiotics, administered intravenously, vasopressors to increase blood pressure, and activated protein C and/or corticosteroids to curb inflammation. These treatment regimens, however, have limited effectiveness.

Because of the association of LPS with sepsis, significant research has focused on vaccinating patients against components of LPS during early stages of the disease (Ziegler E.J. et al., N. Engl. J. Med., 307(20):1225-1230 (1982); Cross A.S. et al., Vaccine, 22(7):812-817 (2004)). One problem with this approach is that *E*. *coli* strains used to generate vaccines contain numerous deleterious genes and proteins that render their use problematic.

Despite the aggressive use of antibiotics and other treatments, sepsis mortality rates remain high. In view of the limited effectiveness and drawbacks of current sepsis treatment regimens, a need exists for compositions and methods for the treatment or prophylaxis of sepsis

### Summary of the Invention

The present invention is defined in the claims. In one embodiment, the present invention relates to pharmaceutical compositions for the prophylaxis of sepsis comprising an effective dose of multiple deletion strain [MDS] bacteria (alternatively referred to herein as a "reduced genome" or a "clean genome" strain) and optionally one or more carriers and/or excipients. The MDS bacteria may be produced by deleting selected genes from a native parental strain of a bacterium or may, for example, be entirely synthesized as an assembly of preselected genes selected to provide a bacterium effective to serve as a therapeutic for the treatment or prevention of sepis. As is readily apparent from the discussion herein, a MDS bacterium has fewer than the full complement of genes found in a native parent strain to which it is compared, and with which it shares certain essential genes. Compositions comprising MDS bacteria lacking genes involved in production of lipoplysaccharide and/or enteric common antigen for the treatment or prophylaxis of sepsis are also provided. In one aspect, the sepsis to be prevented results from exposure to one or more gram-negative bacteria. In a related aspect, the sepsis to be prevented results from exposure to *E. coli* bacteria.

Methods for treating sepsis comprising administering the compositions to a subject having or suspected of having bacteremia are also described. Compositions of the invention may be administered as one component of a combined therapeutic regimen for the treatment of sepsis. In this regard, compositions of the invention may be administered prior to, during or subsequent to the administration of any therapeutic agent directed to the treatment of sepsis. For example, reduced genome bacteria of the invention can be used in combination with vasopressors, activated protein C, corticosteroids, insulin and/or painkillers.

The compositions and methods provide many advantages for the treatment of sepsis over present treatment regimens. For example, MDS bacteria may eliminate many of the problems associated with a sepsis vaccine because many of the deleterious genetic elements found in other gram negative bacteria such as E. *coli* may be removed. Further, components of the bacterial cell wall that contribute to endotoxin/LPS may be removed.

These and other embodiments of the present invention are described in more detail herein below.

### Description of the Drawings

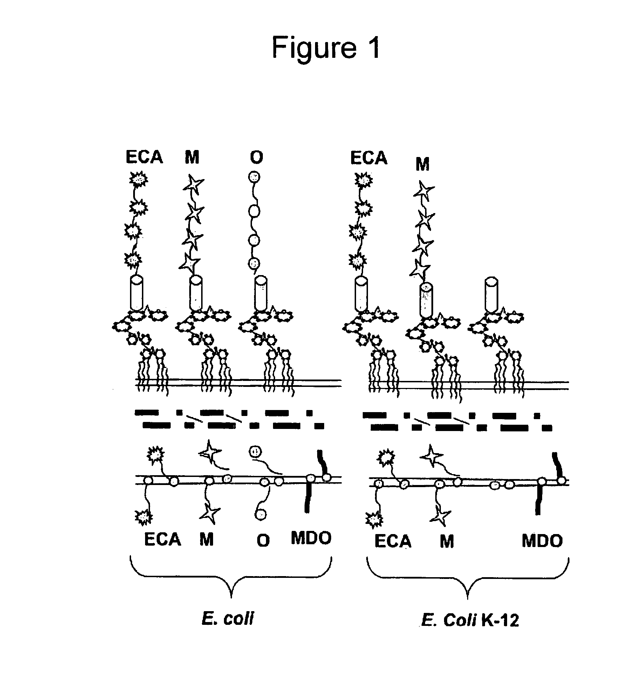

Fig. 1. Schematic of relevant LPS structures and oligosaccharide synthesis at the surface of *E*. *coli.* The nascent oligosaccharides are assembled on an undecaprenyl-phosphate carrier shown as a circle within the inner membrane and then rotated into the periplasm for further processing. LPS is shown with the lipid A, core and oligosaccharide domains indicated. LPS in *E*. *coli* strains in general (left) and in *E. coli* strain K-12, which lacks O-antigen, are depicted.

### Detailed Description of the Invention

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any manner. Embodiments illustrated under any heading may be combined with embodiments illustrated under any other heading.

The use of numerical values in the various ranges specified in this application, unless expressly indicated otherwise, are stated as approximations as though the minimum and maximum values within the stated ranges were both preceded by the word "about." In this manner, slight variations above and below the stated ranges can be used to achieve substantially the same results as values within the ranges. As used herein, the terms "about" and "approximately" when referring to a numerical value shall have their plain and ordinary meanings to one skilled in the pertinent art at issue. Also, the disclosure of ranges is intended as a continuous range including every value between the minimum and maximum values recited as well as any ranges that can be formed by such values. This includes ranges that can be formed that do or do not include a finite upper and/or lower boundary. This also includes ratios that are derivable by dividing a given disclosed numeral into another disclosed numeral. Accordingly, the skilled person will appreciate that many such ratios, ranges, and ranges of ratios can be unambiguously derived from the data and numbers presented herein and all represent various embodiments of the present invention.

The term "multiple deletion strain (MDS) bacteria" herein means a bacteria having about 1% to about 75% of its genome (e.g. protein coding genes) deleted, for example about 5%, about 10%, about 20%, about 30% about 40%, about 50% or about 60% of the genome deleted. In one embodiment, the MDS bacteria used in the practice of the present invention have a genome that is preferably genetically engineered to be at least two percent (2%) and up to twenty percent (20%) (including any number therebetween) smaller than the genome of a native parent strain. Preferably, the genome is at least seven percent (7%) smaller than the genome of a native parent strain. More preferably, the genome is eight percent (8%) to fourteen percent (14%) to twenty percent (20%) (including any number therebetween) or more smaller than the genome of the native parent strain. Alternatively, the genome may be engineered to be less than 20% smaller than the genome of a native parental strain. The term "native parental strain" means a bacterial strain found in a natural or native environment as commonly understood by the scientific community and on whose genome a series of deletions can be made to generate a bacterial strain with a smaller genome. Native parent strain also refers to a strain against which the engineered strain is compared and wherein the engineered strain has less than the full complement of the native parent strain. The percentage by which a genome has become smaller after a series of deletions is calculated by dividing "the total number of base pairs deleted after all of the deletions" by "the total number of base pairs in the genome before all of the deletions" and then multiplying by 100. Similarly, the percentage by which the genome is smaller than the native parent strain is calculated by dividing the total number of nucleotides in the strain with the smaller genome (regardless of the process by which it was produced) by the total number of nucleotides in a native parent strain and then multiplying by 100

In one embodiment, the term "MDS bacteria" refers to bacteria for which removal of the above amounts of genome does not unacceptably affect the ability of the organism to grow on minimal medium. Whether removal of two or more genes "unacceptably affects" the ability of the organism to grow on minimal medium in the present context depends on the specific application. For example, a 30% reduction in proliferation rate may be acceptable for one application but not another. In addition, adverse effect of deleting a DNA sequence from the genome may be reduced by measures such as changing culture conditions. Such measures may turn an otherwise unacceptable adverse effect to an acceptable one. In one embodiment, the proliferation rate is approximately the same as the parental strain. However, proliferation rates ranging from about 5%, 10%, 15%, 20%, 30%, 40% to about 50% lower than that of the parental strain are within the scope of the invention. More particularly, doubling times of bacteria of the present invention may range from about five minutes to about three hours. Non-limiting examples of suitable MDS bacteria, as well as methods for deleting DNA from a bacterium such as *E. coli,* are disclosed in U.S. Pat Nos. 6,989,265 and 7,303,906, U.S. Pat. Pub. Nos. 20060270043, 2006/0199257 and 2007/0054358 and WIPO Pub. No. WO 2003/070880.

Various bacterial strains can be used including, without limitation, *E. coli, Salmonella, Shigella flexneri* and other gram negative bacteria. In a preferred embodiment, the multiple deletion bacteria strain is *E. coli.* Examples of suitable multiple deletion *E. coli* strains include, but are not limited to, MDS39, MDS40, MDS41-R13, MDS41E, MDS42, MDS42msbB, MDS42eca, and MDS66.

It is assumed that at least part of the DNA sequence of the target bacterial strain is available. Preferably, the entire sequence is available. Such complete or partial sequences are readily available in the GenBank database. The full genomic sequences of several strains of *E. coli* have been published (for example, Blattner et al, Science, 277:1453-74, 1997 K-12 Strain MG1655; See also GenBank Accession No. U00096; Pema et al, Nature, 409, 529-533, 2001; Hayashi et al, DNA Res., 8, 11-22,2001, and Welch et al., Proc. Natl. Acad. Sci., USA (2002) 99 (26) 17020-17024 and GenBank Accession No. AE014075), as is the sequence of several other commonly used laboratory bacteria where sequences are found in GenBank.

Various protein coding genes can be deleted to form MDS bacteria. In *E. coli* and other bacteria, a type of DNA sequence that can be deleted includes those that in general will adversely affect the stability of the organism or of the gene products of that organism. Such elements that give rise to instability include without limitation transposable elements, insertion sequences, and other "selfish DNA" elements which may play a role in genome instability. For example, insertion sequence (IS) elements and their associated transposes are often found in bacterial genomes, and thus are targets for deletion. IS sequences are common in *E. coli,* and all of them may be deleted. For purposes of clarity in this document, we use the term IS element and transposable element generically to refer to DNA elements, whether intact or defective, that can move from one point to another in the genome. An example of the detrimental effects of IS elements in science and technology is the fact that they can hop from the genome of the host *E*. *coli* into a BAC plasmid during propagation for sequencing. This artifact could be prevented by deletion from the host cells of all IS elements. For a specific application, other specific genes associated with genomic instability may also be deleted.

MDS bacteria may also be engineered to lack, for example, without limitation, certain genes unnecessary for growth and metabolism of the bacteria, pseudogenes, prophage, undesirable endogenous restriction-modification genes, pathogenicity genes, toxin genes, fimbrial genes, periplasmic protein genes, invasin genes, lipopolysaccharide genes, class III secretion systems, phage virulence determinants, phage receptors, pathogenicity islands, RHS elements, sequences of unknown function and sequences not found in common between two strains of the same native parental species of bacterium. Other DNA sequences that are not required for cell survival can also be deleted or omitted.

Gram-negative bacteria such as *E*. *coli* require at least a minimal structural component of lipopolysaccharide (LPS) for viability and wholesale deletion of genes that encode the pathways required for biosynthesis of these structures is lethal. However, some genes involved in LPS synthesis are not essential, and the cell can tolerate deletion or loss of function of these specific genes.

The lipid A component of LPS is the minimum structure required for viability of gram-negative bacteria. Lipid A is highly conserved among species and is comprised of at least four long-chain fatty acyl groups connected to a pair of modified N-acetyl glucosamine sugars. The long-chain fatty acyl groups may be acylated by secondary fatty acids. The oligosaccharides most proximal to lipid A are referred to as the core, and are serially added onto existing lipid A. The inner core sugars are species specific; the outer core sugars are generally not. Additional oligosaccharides attached to the outer core sugars are synthesized on membrane embedded undecaprenyl-phosphate carriers and are added to the maturing LPS as pre-formed repeating units. These pre-formed repeating oligosaccharides include the O-antigen, the M-antigen (colanic acid) and the enteric common antigen (ECA). *See* Figure 1.

In one embodiment, the MDS bacteria lack one or more genes implicated in or necessary for production of a lipopolysaccharide. Genes implicated in and/or necessary for production of a lipopolysaccharide are known in the art, for example as disclosed in Reeves, P and Wang, L (2002). Genomic organization of LPS-specific loci. Curr Top Microbiol Immunol 264 (1): 109-35 and Patil, P et al., (2004). Variation suggestive of horizontal gene transfer at a lipopolysaccharide (Ips) biosynthetic locus in Xanthomonas oryzae pv. oryzae, the bacterial leaf blight pathogen of rice. BMC Microbiol 4 (1): 40, each of which are hereby incorporated by reference herein.

In a preferred embodiment, candidate genes for deletion include one or more genes implicated in or necessary for the biosynthesis of the O-antigen and/or the M-antigen (colanic acid) of the lipopolysaccharide. *E. coli* K-12 derivatives are incapable of completely synthesizing O-antigen due to a disruption of the *wbbL* gene by IS5. However, this strain remains competent to produce other oligosaccharides such as M-antigen, the gene cluster of which is co-localized with many of those involved in O-antigen biosynthesis. The *rfa* gene cluster between nucleotide 3792010 and 3806121 of the *E. coli* chromosome may be deleted. Deletion of the *rfa* gene cluster appears to delete both the O- and M-antigen oligosaccharides. Illustrative genes implicated in or necessary for production of lipopolysaccharide include the *rfaI, rfaJ, rfe* genes, which may be deleted individually or in combination.

In another preferred embodiment, the MDS bacteria comprises deletions of one or more genes associated with production of the enteric common antigen (ECA), a repeating trisaccharide carbohydrate moiety common to all *Enterobacteriaceae,* some of which is associated with LPS. The ECA genes are generally clustered on the E. *coli* chromosome between nucleotide 3965939 and 3980295 and comprise 13 genes between *rfe* and *yifK.*

In a particularly preferred embodiment, the MDS bacteria lack a functional *msbB* gene. *MsbB* encodes myristoyl acyltransferase which catalyzes O-acyl attachment of β-hydroxy myristate to primary β-hydroxy myristoyl moieties of lipid A (i.e., secondary acylation). Deletion of this gene produces a cell with a 1000-10,000-fold reduction in the ability to stimulate TNFα by human tissue culture cells (Sommerville et al., J. Clin. Invest. 1996 97:359). However, MG1655 *msbB* mutants do not appear to have reduced endotoxin levels as measured by the kinetic chromogenic *Limulus* amaebocyte lysate (CLAL) assay (unpublished observation). In a preferred embodiment, the MDS bacteria lack a functional *msbB* gene.

In another preferred embodiment, the MDS bacteria lack one or more genes involved in the biosynthesis of the outer core of LPS. Illustrative genes involved in the biosynthesis of the outer core of LPS include, without limitation, *waaL, waaV, waaW, waaY* and *waaT.*

In various embodiments, the present invention relates to methods for the prevention of sepsis. Subjects in need of such treatment include those at risk for or suffering from the presence of a pathogen in the blood such as a bacterial pathogen (bacteremia) or a viral pathogen. Subjects particularly able to benefit from the present invention are those at risk for or suffering from infection by gram-negative bacteria, particularly *E. coli.* Subjects at risk for sepsis include those suffering burns, gunshot wounds, renal or hepatic failure due to chemical poisoning or abuse, and the like.

In one embodiment, the present invention relates to methods for preventing sepsis in a subject in need of such treatment, comprising administering to the subject a therapeutically effective amount of multiple deletion strain bacteria. The multiple deletion strain bacteria may be administered as live bacteria and may be attenuated by the inactivation of one or more virulence factors. Alternatively, the multiple deletion strain bacteria may be administered as killed bacteria.

In one aspect, the present invention contemplates the administration of compositions comprising multiple deletion strain bacteria to a subject prior to the onset of symptoms (e.g. prophylactically). In particular, the present invention contemplates the administration of compositions comprising multiple deletion strain bacteria to subjects at high risk for sepsis, including, without limitation, surgical patients, cancer patients undergoing chemotherapy and/or radiation therapy (especially patients with acute leukemia, chronic lymphocytic leukemia, multiple myeloma, Hodgkin's disease, or non-Hodgkin's lymphoma), burn victims, trauma patients, and patients with any pathological condition which results in the introduction of bacteria into the bloodstream. Other subjects at high risk for developing sepsis include, without limitation, patients with one or more of the following conditions, each of which may be an underlying cause of sepsis: congenital microvillous atrophy; corticobasal degeneration; Deal-Barratt-Dillon syndrome; Febrile Ulceronecrotic Mucha-Habermann disease; Fransicsella tularenis infection; streptococcal infection; histoplasmosis; listeriosis; noma; pancreatic abscess; sickle cell anemia; Simpson-Golabi-Behmel syndrome, type 2; subphrenic abscess; toxic epidermal necrolysis; and Waterhouse-Friederischsen syndrome. Additional subjects at high risk for developing sepsis include, without limitation, subjects with upper urinary tract infections, subjects with bacterial pneumonia and subjects with skin infections (e.g. cellulitis). Additional subjects at high risk for developing sepsis include, without limitation, subjects with peritonitis and subjects with inflammatory bowel disease (Crohn's disease and/or ulcerative colitis). Subjects with any of the aforementioned conditions may be administered a prophylactically effective amount of a composition comprising multiple deletion strain bacteria in order to prevent sepsis.

The present disclosure contemplates the administration of compositions comprising multiple deletion strain bacteria to a subject after a pathogen has been detected (e.g. bacteremia) and/or sepsis is suspected. Evidence of bacteremia may include (1) core temperature higher than 38° C or lower than 35° C (2) peripheral blood leukocyte count greater than 12x10⁹/L or less than 3x10⁹/L (3) growth of gram-negative bacteria from a blood culture or (4) documented or suspected site of gram-negative infection.

In another aspect, the present disclosure contemplates the administration of compositions comprising multiple deletion strain bacteria to a subject with sepsis. A systemic septic reaction is characterized by at least one of the following (1) arterial hypotension (systolic blood pressure < 90mm hg or an acute drop of 30 mm Hg) (2) metabolic acidosis (base deficit > 5 mEq/L) (3) decreased systemic vascular resistance (systemic vascular resistance < 800 dynes/s/cm⁵) (4) tachypnea (respiratory rate > 20/min or ventilation > 10 L/min if mechanically ventilated) or (5) otherwise unexplained kidney dysfunction (urine output < 30 ml/h) or lungs. Preferably, compositions of the invention are administered to a subject prior to a systemic infection, if possible.

Described herein are methods for treating and/or preventing one or more of the symptoms of sepsis comprising administering to a subject in need of such treatment, a therapeutically effective amount of multiple deletion strain bacteria. Symptoms of sepsis that can be prevented or treated according to the present invention include, without limitation, fever, hypotension, neutropenia, luekopenia, thrombocytopenia, shock, and multiple organ failure.

Described herein are methods for treating and/or preventing systemic inflammation comprising administering to a subject in need of such treatment, a therapeutically effective amount of multiple deletion strain bacteria. Systemic inflammation that may be treated or prevented with compositions of the invention may be mediated by damage-associated molecular profiles (DAMPS), as released during cell damage or disease, or may be mediated by pathogen-associated molecular profiles (PAMPS) as released during infection with a pathogen such as bacteria (e.g. bacteremia) or a viral pathogen. Without wishing to be bound by theory, it is expected that administration of compositions of the invention may protect against DAMP-mediated systemic inflammation by inducing low-level PAMP activation by an otherwise innocuous multiple deletion strain bacteria such as MDS66.

Described herein are methods for treating and/or preventing one or more conditions associated with systemic inflammation comprising administering to a subject in need of such treatment, a therapeutically effective amount of multiple deletion strain bacteria. Systemic inflammation that may be treated or prevented with compositions of the invention may be mediated by damage-associated molecular profiles (DAMPS), as released during cell damage or disease, or may be mediated by pathogen-associated molecular profiles (PAMPS) as released during infection with a pathogen such as bacteria (e.g. bacteremia) or a viral pathogen.

In an embodiment, the present invention provides a composition for use in a method comprising administering an effective amount of multiple deletion strain bacteria, wherein said multiple deletion strain bacteria is an *Escherichia coli* multiple deletion strain, as defined in the claims.

In a related embodiment, the present invention provides a composition for use in a method comprising administering an effective amount of *E. coli* multiple deletion strain bacteria, wherein said *E. coli* multiple deletion strain bacteria lack one or more genes involved in production of lipopolysaccharide, as defined in the claims. In a preferred embodiment, the *E. coli* multiple deletion strain lacks a functional *msbB* gene. In another preferred embodiment, the *E. coli* multiple deletion strain lacks one or more genes selected from the group consisting of *rfaI, rfaJ* and *rfe*

In a related embodiment, the present invention provides a composition for use in a method comprising administering an effective amount of *E. coli* multiple deletion strain bacteria, wherein said *E. coli* multiple deletion strain bacteria lack one or more genes involved in production of enteric common antigen, wherein said one or more genes are located on the *E. coli* chromosome between nucleotide 3965939 and 3980295, as defined in the claims.

In one instance, a therapeutically effective amount of multiple deletion strain bacteria is administered to a subject as one component of a combined therapeutic regimen for the treatment of sepsis. In this regard, compositions of the invention may be administered prior to, during or subsequent to the administration of one or more additional therapeutic agents directed to the treatment of sepsis. For example, reduced genome bacteria of the invention can be used in combination with vasopressors, activated protein C, corticosteroids, insulin, painkillers and the like. In a related embodiment, administration of compositions of the invention to a subject may be followed by treatment with an antibiotic, typically an aminoglycoside such as gentamycin or a beta-lactam such as penicillin, cephalosporin and the like. Administration of an antibiotic may occur within about 24 hours, within about 48 hours, within about 72 hours, within about 96 hours or anywhere therebetween, but will generally occur after one or more symptoms of sepsis have ameliorated in the subject.

Protein C is a vitamin K-dependent serine protease produced in an inactive form by the liver after which it circulates in the plasma and is activated by thrombin at the surface of endothelial cells. When so activated, protein C inhibits coagulation. Activated Protein C formulations are approved by the FDA for administration to patients with sepsis and marketed by Eli Lilly under the tradename XIGRIS™.

Therapeutic compositions of the invention may contain, in addition to a therapeutically effective amount of multiple deletion strain bacteria, one or more pharmaceutically acceptable carriers and/or excipients, such as water, bacterial culture fluid, glycerol, and phosphate buffered saline.

Therapeutic compositions of the invention may be administered to animals for veterinary use, such as with domestic animals, and clinical use in humans, in a manner similar to other therapeutic agents. For example, therapeutic compositions of the invention may be administered, without limitation, to chickens, turkeys, pigs, sheep, cattle, goats, buffalo, primates, dogs, cats, horses and mice. In one embodiment, therapeutic compositions of the invention may be administered to protect against sepsis resulting from primary or secondary coliform infections in birds such as chickens and turkeys, particularly young chickens and turkeys. Coliform infections (and the resulting symptoms) are often caused by strains of *E. coli.*

Compositions of the invention comprising multiple deletion strain bacteria may be administered intravenously, intramuscularly, intraperitoneally, intrathecally, orally, anally, or the like. The term "unit dose" when used in reference to therapeutic compositions of the invention refers to physically discrete units suitable as unitary dosage for humans, each unit containing a predetermined quantity of multiple deletion strain bacteria calculated to produce the desired therapeutic effect. The multiple deletion strain bacteria can also be administered to the subject via catheter or infusion pump, set to deliver the desired dosage over a period of time.

Compositions of the invention are to be administered in a manner compatible with the dosage formulation, and in a therapeutically effective amount. The quantity to be administered depends on the subject to be treated. Precise amounts of bacteria required to be administered depend on the judgment of the practitioner and may be peculiar to each individual. However, suitable dosage ranges are of the order of 10³ and 10¹⁵ bacteria and depend on the route of administration. It will be noted that in general, therapeutic doses will likely be higher than doses required in prophylactic treatment. In treating a subject to inhibit or reverse sepsis, the subject's clinical signs are measured regularly and the dosage of bacteria adjusted accordingly. For example, frequent measurement of the subject's fibrin degradation product and fibrinogen levels may be monitored. Measurement of changes of vital signs and in white cell count over time may also be monitored. In situations where sepsis has not yet become clinically apparent but is expected to occur, such as in the first several hours of a serious injury, administration of compositions of the invention may be initiated. In situations where sepsis has become clinically apparent, compositions of the invention are administered such that at least one symptom of sepsis is reduced.

The phrase "therapeutically effective amount" or "prophylactically effective amount" is used herein to mean an amount sufficient to treat or prevent one or more conditions associated with sepsis.

Compositions of the invention may be administered to patient once or may be administered to a patient repeatedly over a period of time. For example, a patient at risk for developing sepsis may be administered one dose after which his or her condition may be monitored. Alternately, patients may be administered multiple doses for an additional period of time. The additional period of time may be 1 to about 4 weeks. For example, a patient may be administered two doses during a one week interval. Administration of compositions of the invention to a patient may be daily, bidaily, weekly, bi-weekly, monthly, bi-monthly, *etc.*

### Example 1

### Production of Reduced Genome E. coli

Reduced genome strain MDS39 was produced as described in International Patent Publication No. WO 2003/070880, which is incorporated herein by reference. Briefly, a series of reduced genome strains (MDS01-MDS39) were produced by making a series of 39 cumulative deletions (approximately 14.1 % of the genome) of nucleic acid sequences from the parental strain *E. coli* MG1655.

Hybridization to genome scanning chips (NimbleGen Systems, Madison, WI) containing the K-12 sequence and all sequences in the IS database revealed that MDS39, the first strain designed to lack all IS elements, unexpectedly contained additional copies of IS elements that had hopped to new locations during its production. These IS elements were deleted to produce MDS40. *The fhuACDB* (the *tonA* locus) was deleted from MDS40 to produce MDS41. Strains lacking the *tonA* locus are resistant to infection by bacteriophage T1, a common laboratory scourge. The location and function of each cumulative deletion made to produce MDS01-MDS41 can be found at Table 2 of U.S. Application Publication No. 2007/0054358, the entire content of which is incorporated herein by reference. The *endA* gene was deleted from MDS41 to produce MDS42. MDS43 was produced by deleting an additional 45 kb covering the *lac* operon from parental strain MDS42. Some genome statistics of MG1655 and related multiple deletion strains are provided in Table 1.

More than 30 genes involved in colanic acid production were deleted from MDS21 to produce MDS22. Accordingly, MDS22, MDS23...MDS66 lack colanic acid.

In *E. coli,* LPS (endotoxin) is modified by addition of oligosaccharides which contribute strongly to its antigenic properties. MsbB catalyzes O-acyl attachment of β-hydroxy myristate to the primary β-hydroxy myristoyl moiety of lipid A. Loss of this enzymatic activity limits LPS to the penta-acyl form, which is less immunogenic. The entire LPS cannot be removed as the core lipid A is essential for outer membrane integrity. The *msbB* gene was deleted from MDS42 to produce MDS42*msbB.*

ECA is an antigenic glycolipid found in the outer membrane of all gram-negative bacteria. Thirteen contiguous genes encoding enzymes required for ECA biosynthesis were deleted from MDS42 to produce MDS42*eca*.

Strain MDS42*msb*/*eca* was produced by deleting the *msbB* gene as well as the thirteen contiguous genes encoding enzymes required for ECA biosynthesis from MDS42.

MDS66 was produced by deleting several genes from MDS42. Approximately 19.2% of the genome has been deleted in MDS66. MDS66 comprises an intact *msbB* gene as well as the thirteen contiguous genes encoding enzymes required for ECA biosynthesis. Genes (identified by "b" numbers based on the designations set out in Blattner et al., Science, 277:1453-74 and in GenBank Accession No. 400096) deleted from each deletion strain from MDS 12 to MDS73 may be found at Tables 8 and 9 of U.S. Application Publication No. 2006/0199257, the entire content of which is incorporated herein by reference.

**Table 1: Genome statistics of MG1655 and related multiple deletion strains**

| | MG1655 | MDS12 | MDS41 | MDS42 | MDS43 |
|---|---|---|---|---|---|
| Total no. genes | 4444 | 4029 | 3743 | 3742 | 3704 |
| Genome size (bp) | 4639675 | 4263492 | 3977067 | 3976359 | 3931408 |
| Replichore imbalance (bp) | 30517 | 141360 | 139331 | 138623 | 183574 |
| Total no. genes deleted | 0 | 415 | 701 | 702 | 740 |
| Total bp DNA deleted | 0 | 376183 | 662608 | 663316 | 708267 |
| % genome deleted | 0 | 8.11% | 14.28% | 14.30% | 15.27% |

### Example 2

### Preparation of Bacteria

Cultures for immunization and challenge (see Examples 3 and 4) were prepared identically. 20 ml cultures in LB were inoculated from freezer stocks of each bacteria and grown overnight (about 17 hours) at 30 °C. Turbidity was measured (optical density (OD) at 600 nm) at the harvest point. 10 ml culture was centrifuged at 2500 x g at 4 °C for 15 minutes. The resulting bacterial pellet was then resuspended in 10 mls of ice-cold sterile 1x PBS and bacteria were collected by centrifugation as before. Bacteria were then resuspended in 5 mls ice-cold sterile 1x PBS, 15% glycerol and the turbidity measured. Suspensions were adjusted to an OD₆₀₀ of approximately 1.1 by further addition of PBS-glycerol, then 0.3 ml aliquots were dispensed into 0.65 ml microfuge tubes and frozen at -80 °C. After several hours of freezing, one tube of each strain was thawed for 2 minutes at 37 °C and serial dilutions made. 0.1 ml samples were plated on LB agar to determine titers of viable colony-forming bacteria. For control immunizations, 0.3 ml of sterile PBS-glycerol were used.

### Example 3

### Clean Genome Bactofection of Mutant Stx2A

Reduced genome strain MDS42 carrying a plasmid encoding a mutant Stx2A gene (mStx2A) was tested for the ability to act as a bacterial vaccine carrier. Briefly, the *stx2A* gene encodes the Shiga toxin active site subunit. Beginning with the enterohemorrhagic E. *coli* (EHEC) O157:H7 strain EDL933 (sequenced in the Blattner lab at the University of Wisconsin), mutations were generated on opposite sides of the active site pocket. The mutations were shown to eliminate the protein's toxic glycosylase activity without affecting its immunogenicity. mStx2A was placed under the control of a CMV promoter in a plasmid vector which also comprises both a single copy and an inducible multicopy replicon, the *invA* gene from *Yersinia pseudotuberculosis,* enabling the MDS42 bacteria to invade certain mammalian cell types.

To assay for delivery of reporter DNA, female Balb/c mice about 6-8 weeks old were initially immunized by either oral gavage (feeding tube) or intraperitoneal (IP) injection of either (1) MDS42 carrying the plasmid vector with a lacZ reporter gene (encoding B-galactosidase) under the control of the CMV promoter in place of mStx2A or (2) DH10B strain carrying the same plasmid. Unexpectedly, all the mice that were injected IP with DH10B died within 3 days whereas 90% of the mice injected with similar doses of MDS42 survived. At weekly intervals after immunization, blood samples were collected and the sera tested for anti-β-galactosidase antibodies by ELISA. No antibodies were detected (5 experiments, total of 25 mice plus controls).

Next, female Balb/c mice about 6-8 weeks old were immunized by either oral gavage or IP injection of MDS42 bacteria carrying the plasmid with mStx2A under the control of the CMV reporter. The mice were then challenged with the lowest dose of toxin that was predicted to kill untreated (naive) mice. Mice injected IP with MDS42 carrying the mStx2A plasmid were successfully protected against challenge with Shiga toxin. Surprisingly, control immunizations of MDS42 without the mStx2A vaccine plasmid were also protected against lethal challenge with Shiga toxin. Even allowing for the ~20% survival of unimmunized mice, these are striking results. In comparison, the gavage-delivered DNA vaccine protected about 27% of the mice (57% corrected for 20% survival of naive mice). Results are provided at Table 2 below:

**Table 2**

| **Delivery mode** | **Plasmid¹** | **Stx survivors** | **Stx % survivors** | **Naive survivors** | **Naïve % survivors** | **Number of experiments** |
|---|---|---|---|---|---|---|
| Injected | mStx2A | 19/19 | 100 | 4/19 | 21 | 4 |
| | mStx2A | 10/10 | 100 | 2/10 | 20 | 2 |
| | mStx2A | 8/8 | 100 | 2/10 | 20 | 2 |
| | mStx2A | 4/4 | 100 | 1/4 | 25 | 1 |
| | none | 12/15 | 75 | 4/19 | 21 | 4 |
| Gavage | mStx2A | 8/14 | 57 | 3/15 | 20 | 4 |
| | mStx2A | 4/7 | 57 | 2/10 | 20 | 2 |
| | mStx2A | 2/10 | 20 | 2/10 | 20 | 2 |
| | mStx2A | 2/5 | 40 | 2/8 | 25 | 1 |
| | none | 4/17 | 23 | 4/19 | 21 | 4 |

| | | | | | | |
|---|---|---|---|---|---|---|
| ¹ mStx2A plasmid contains mStx2A under the control of the CMV promoter and invasin under its native control | | | | | | |

### Example 4

### Toxicity of E. coli Strains by Intraperitoneal Injection Into Mice

A mouse model for sepsis was developed. Mice received an intraperitoneal injection of either wild type *E*. *coli* strains (DH10B or MG1655) or reduced genome *E. coli* strains (MDS12, MDS21, MDS22, MDS42, MDS42*eca*, MDS42*msbB,* MDS42*msbB*/*eca,* or MDS66) in PBS-glycerol, prepared as described in Example 2. The viable bacteria/dose and OD₆₀₀/dose for each strain is displayed at Table 3. One mouse received an intraperitoneal injection of 0.3 ml of sterile PBS/glycerol as a control. As demonstrated at Table 3, injection of unmodified *E. coli* strains into mice resulted in 85% (MG1655) and 90% (DH10B) mortality within 3 days consistent with sepsis. Injection of reduced genome *E*. *coli* strains, unexpectedly, resulted in little to no mortality in the case of MDS42, MDS42*msbB,* MDS42*msbB*/*eca* and MDS66. Reduced genome strain MDS12, however, resulted in 100% mortality and reduced genome strains MDS21 and MDS22 resulted in 90% mortality.

**Table 3**

| MDS Strain | Mortality (deaths/# mice) | Viable bacteria/dose | OD₆₀₀/dose | Mortality % |
|---|---|---|---|---|
| MDS12 | 10/10 | 8.8 x 10⁸ | 0.14 | 100 |
| MDS21 | 9/10 | | | 90 |
| MDS22 | 9/10 | 5 x 10⁸ | | 90 |
| MDS42 | 1/10 | 4.1 x 10⁸ | 0.12 | 10 |
| MDS42-*eca* | 4/10 | 3.6 x 10⁸ | 0.09 | 40 |
| *MDS42-msbB* | 1/10 | 4.3 x 10⁸ | 0.16 | 10 |
| MDS42-*msbB*/*eca* | 1/10 | 7.0 x 10⁸ | 0.25 | 10 |
| MDS66 | 0/10 | 3.3 x 10⁹ | 0.09 | 0 |
| DH10B | 18/20 | 4.8 x 10⁸ | 0.16 | 90 |
| MG1655 | 17/20 | 6.9 x 10⁸ | 0.11 | 85 |
| PBS/glycerol | 0/10 | 0 | 0 | 0 |

### Example 5

### Treatment of Sepsis by Administration of Reduced Genome Bacteria

A mouse model was used to investigate the therapeutic potential of multiple deletion strain bacteria in septic death accompanying bacteremia. Mice were separated into two groups. On day 0, mice from Group One received a first intraperitoneal injection of multiple deletion *E. coli* strain MDS66 (OD₆₀₀ of 1.14; 6.0 x 10⁸ viable cells) while mice from Group Two received an intraperitoenal injection of PBS-glycerol. Six days later, mice from Group One received another intraperitoneal injection of the same strain at the same dose while mice from Group Two received another injection of PBS-glycerol. On day fourteen, eight mice from Group One and five mice from Group Two were challenged with a single injection of live wild type bacteria from the *E*. *coli* strain DH10B (OD₆₀₀ of 1.12; 3.1 x 10⁸ viable cells). As seen in Table 4, the intraperitoneal injection of live DH10B caused death in 80% (experiment 1) and 100% (experiment 2) of the mice from Group Two within 4 days of receiving the injection. Surprisingly, all of the mice from Group One survived. Moreover, these surviving mice remained alive and healthy. Thus, delivering live MDS66 bacteria was able to protect mice from a lethal challenge of live bacteria from the strain DH10B. This indicates that injection of reduced genome bacteria strains provides an effective treatment for and prevention of septic death in mammals with gram-negative bacteremia. Without wishing to be bound by theory, multiple deletion strain bacteria such as MDS66 may avoid full immune recognition but may be effective enough to attenuate the broad inflammatory response typical of sepsis (PAMP-mediated systemic inflammation) or extensive tissue damage (DAMP-mediated systemic inflammation). It is expected that multiple deletion strain bacteria comprising deletions of at least the DNA segments deleted from MDS42 will provide effective treatment and/or prevention of septic death in mammals with gram-negative bacteremia.

**Table 4: Challenge of naïve mice with DH10B**

| Experiment | Final OD₆₀₀ | Viable count/dose | Mortality - Group One | Mortality - Group Two |
|---|---|---|---|---|
| 1 | 1.0 | 3.3 x 10⁸ | 0/10 | 8/10 |
| 2 | 0.66 | 4.8 x 10⁸ | 0/10 | 10/10 |

### Example 6

### Treatment of Sepsis in Human Subjects by Administration of Reduced Genome Bacteria

Multiple deletion strain bacteria may be used prophylactically or therapeutically to treat sepsis in humans. Individuals at risk of contracting sepsis, particularly trauma patients or patients undergoing surgery, or those with sepsis may be administered a therapeutically or prophylactically effective dose of multiple deletion strain bacteria to prevent or reduce the severity of the disease. Therapeutically the dose may be administered repeatedly until remission of the disease is apparent. The preferred route of administration is intravenous administration.

## Claims

1. A composition for use in a method of preventing sepsis in an animal, comprising an effective amount of multiple deletion strain *Escherichia coli* bacteria, wherein said multiple deletion strain bacteria have a genome that is genetically engineered to be at least 5% at least 7%, at least 14% or at least 19% smaller than the genome of a native parent strain,
and wherein said multiple deletion strain bacteria comprise a deletion of one or more genes selected from the group consisting of: b2190; b2191; b2192; b3215; b3216; b3217; b3218; b3219; b3504; b3505; b1070; b1071; b1072; b1073; b1074; b1075; b1076; b1077; b1078; b1079; b1080; b1081; b1082; b1083; b1878; b1879; b1880; b1881; b1882; b1883; b1884; b1885; b1886; b1887; b1888; b1889; b1890; b1891; b1892; b1893; b1894; b1917; b1918; b1919; b1920; b1921; b1922; b1923; b1924; b1925; b1926; b1927; b1928; b1929; b1930; b1931; b1932; b1933; b1934; b1935; b1936; b1937; b1938; b1939; b1940; b1941; b1942; b1943; b1944; b1945; b1946; b1947; b1948; b1949; b1950; b4324; b4325; b4326; b4327; b4328; b4329; b4330; b4331; b4332; b4333; b4334; b4335; b4336; b4337; b4338; b4339; b4340; b4341; b4342; b4345; b4346; b4347; b4348; b4349; b4350; b4351; b4352; b4353; b4354; b4355; b4356; b4357; b4358; b4486; b0497; b0498; b0499; b0500; b0501; b0502; b0700; b0701; b0702; b0703; b0704; b0705; b0706; b1456; b1457; b1458; b1459; b1460; b1461; b1462; b3481; b3482; b3483; b3484; b3592; b3593; b3594; b3595; b3596; b0981; b0982; b0983; b0984; b0985; b0986; b0987; b0988; b1021; b1022; b1023; b1024; b1025; b1026; b1027; b1028; b1029; b2080; b2081; b2082; b2083; b2084; b2085; b2086; b2087; b2088; b2089; b2090; b2091; b2092; b2093; b2094; b2095; b2096; b4438; b3440; b3441; b3442; b3443; b3444; b3445; b4451; b3556; b3557; b3558; b4455; b1786; b0150; b0151; b0152; and b0153 of the E. coli K-12 strain MGI655.

2. The composition for use according to claim 1, wherein said multiple deletion strain bacteria have deleted therefrom at least the DNA segments deleted from MDS42.

3. The composition for use according to claim 1, wherein said multiple deletion strain bacteria is MDS42.

4. The composition for use according to claim 1, wherein said multiple deletion strain bacteria is MDS66.

5. The composition for use according to any one of the preceding claims, wherein said effective amount of multiple deletion strain bacteria is to be administered prior to, during, or subsequent to an additional treatment regimen.

6. The composition for use according to any one of the preceding claims, wherein said multiple deletion strain bacteria are live multiple deletion strain bacteria.

7. The composition for use according to any one of the preceding claims, wherein said *E. coli* multiple deletion strain lacks a functional *msbB* gene.

8. The composition for use according to any one of the preceding claims, wherein said multiple deletion strain lacks one or more genes involved in production of lipopolysaccharide, wherein said one or more genes is located in the rfa gene cluster between nucleotide 3792010 and 3806121 of the *E*. coli chromosome; and
wherein optionally said one or more genes is selected from the group consisting of *rfaI, rfaJ* and *rfe* of combinations thereof.

9. The composition for use according to any one of the preceding claims, wherein said multiple deletion strain lacks one or more genes involved in production of enteric common antigen, wherein said one or more genes are located on the E. coli chromosome between nucleotide 3965939 and 3980295.

10. The composition for use according to any one of the preceding claims, wherein in said method:
(a) said composition is to be administered to the animal in a single dose;
(b) multiple doses of said composition are to be administered to the animal; or
(c) multiple doses of said composition are to be administered to the animal and said composition is administered weekly.

11. The composition for use according to any one of the preceding claims, wherein in said method said animal is a human.

12. The composition for use according to any one of the preceding claims, wherein the said multiple deletion strain bacteria have a genome that is genetically engineered to be at least 5% and up to 20% smaller than the genome of a native parent strain.

13. The composition for use according to any one of the preceding claims, wherein said method is a method of preventing sepsis caused by exposure to gram-negative bacteria.

14. The composition for use according to claim 13, wherein in said method the gram-negative bacteria is *E.coli.*

15. A pharmaceutical composition comprising multiple deletion strain bacteria and a pharmaceutically acceptable carrier, wherein said multiple deletion strain bacteria have a genome that is genetically engineered to be at least 5%, at least 7%, at least 14% or at least 19% smaller than the genome of its native parent strain;
and wherein optionally said multiple deletion strain bacteria is MDS42 or MDS66.

## Patentansprüche

1. Zusammensetzung zur Verwendung in einem Verfahren zur Prävention von Sepsis bei einem Tier, das eine wirksame Menge *Escherichia*-*coli*-Bakterien eines Stammes mit mehreren Deletionen umfasst, worin die Bakterien eines Stammes mit mehreren Deletionen ein Genom aufweisen, das gentechnisch so manipuliert wurde, dass es um zumindest 5 %, um zumindest 7 %, um zumindest 14 % oder um zumindest 19 %, kleiner ist als das Genom eines nativen Ausgangsstammes
und worin die Bakterien eines Stammes mit mehreren Deletionen eine Deletion eines oder mehrerer aus der aus b2190; b2191; b2192; b3215; b3216; b3217; b3218;b3219;
**b3504; b3505; b1070; b1071; b1072; b1073; b1074; b1075; b1076; b1077; b1078; b1078; b1079; b1080**; **b1081**; **b1082; b1083; b1878; b1879; b1880; b1881; b1882; b1883; b1884**, **b1885; b1886; b1887; b1888; b1889; b1890; b1891; b1892; b1893; b1894; b1917; b1918; b1919; b1920; b1921; b1922; b1923; b1924; b1925; b1926; b1927; b1928; b1929; b1930; b1931; b1932; b1933; b1934; b1935; b1936; b1937; b1938; b1939; b1940; b1941; b1942; b1943; b1944; b1945; b1946; b1947; b1948; b1949; b1950; b4324; b4325; b4326; b4327; b4328; b4329; b4330; b4331; b4332; b4333; b4334; b4335; b4336; b4337; b4338; b4339; b4340; b4341; b4342; b4345; b4346; b4347; b4348; b4349; b4350; b4351; b4352; b4353; b4354; b4355; b4356; b4357; b4358; b4486, b0497; b0498; b0499; b0500; b0501; b0502; b0700; b0701; b0702; b0703; b0704; b0705; b0706; b1456; b1457; b1458; b1459; b1460; b1461; b1462; b3481; b3482; b3483; b3484; b3592; b3593; b3594; b3595; b3596; b0981; b0982; b0983; b0984; b0985; b0986; b0987; b0988; b1021; b1022; b1023; b1024; b1025; b1026; b1027; b1028; b1029; b2080; b2081; b2082; b2083; b2084; b2085; b2086; b2087; b2088; b2089**; **b2090; b2091; b2092; b2093; b2094; b2095; b2096; b4438; b3440; b3441; b3442; b3443;** b3444; b3445; b4451; b3556; b3557; b3558; b4455; b1786; b0150; b0151; b0152; und b0153 des E.-*coli*-K12-Stammes MGI655 bestehenden Gruppe ausgewählter Gene umfasst.

2. Zusammensetzung zur Verwendung nach Anspruch 1, worin aus den Bakterien eines Stammes mit mehreren Deletionen zumindest die aus MDS42 deletierten DNA-Segmente deletiert sind.

3. Zusammensetzung zur Verwendung nach Anspruch 1, worin die Bakterien eines Stammes mit mehreren Deletionen MDS42 sind.

4. Zusammensetzung zur Verwendung nach Anspruch 1, worin die Bakterien eines Stammes mit mehreren Deletionen MDS66 sind.

5. Zusammensetzung zur Verwendung nach einem der vorangegangenen Ansprüche, worin die wirksame Menge der Bakterien eines Stammes mit mehreren Deletionen vor, während oder nach einem zusätzlichen Behandlungsprogramm verabreicht werden.

6. Zusammensetzung zur Verwendung nach einem der vorangegangenen Ansprüche, worin die Bakterien eines Stammes mit mehreren Deletionen lebende Bakterien eines Stammes mit mehreren Deletionen sind.

7. Zusammensetzung zur Verwendung nach einem der vorangegangenen Ansprüche, worin dem *E*.*-coli*-Stamm mit mehreren Deletionen ein funktionelles msbB-Gen fehlt.

8. Zusammensetzung zur Verwendung nach einem der vorangegangenen Ansprüche, worin dem Stamm mit mehreren Deletionen ein oder mehrere Gene fehlen, die an der Produktion von Lipopolysacchariden beteiligt sind, worin das eine oder die mehreren Gene sich im rfa-Gencluster zwischen den Nucleotiden 3792010 und 3806121 des E.-*coli*-Chromosoms befinden; und
worin gegebenenfalls das eine oder die mehreren Gene aus der aus rfal, rfaJ und rfe oder Kombinationen daraus bestehenden Gruppe ausgewählt sind.

9. Zusammensetzung zur Verwendung nach einem der vorangegangenen Ansprüche, worin dem Stamm mit mehreren Deletionen ein oder mehrere Gene fehlen, die an der Produktion von Enteric Common Antigen beteiligt sind, worin das eine oder die mehreren Gene sich im *E.-coli*-Chromosom zwischen den Nucleotiden 3965939 und 3980295 befinden.

10. Zusammensetzung zur Verwendung nach einem der vorangegangenen Ansprüche, worin in dem Verfahren
(a) die Zusammensetzung dem Tier in einer Einzeldosis zu verabreichen ist;
(b) mehrere Dosen der Zusammensetzung dem Tier zu verabreichen sind; oder
(c) mehrere Dosen der Zusammensetzung dem Tier zu verabreichen sind und die Zusammensetzung wöchentlich verabreicht wird.

11. Zusammensetzung zur Verwendung nach einem der vorangegangenen Ansprüche, worin in dem Verfahren das Tier ein Mensch ist.

12. Zusammensetzung zur Verwendung nach einem der vorangegangenen Ansprüche, worin die Bakterien eines Stammes mit mehreren Deletionen ein Genom aufweisen, das gentechnisch so manipuliert wurde, dass es um zumindest 5 % und bis zu 20 % kleiner ist als das Genom eines nativen Ausgangsstammes.

13. Zusammensetzung zur Verwendung nach einem der vorangegangenen Ansprüche, worin das Verfahren ein Verfahren zur Prävention von Sepsis ist, die durch Kontakt mit gramnegativen Bakterien ausgelöst wird.

14. Zusammensetzung zur Verwendung nach Anspruch 13, worin in dem Verfahren das gramnegative Bakterium *E*. *coli* ist.

15. Pharmazeutische Zusammensetzung, die Bakterien eines Stammes mit mehreren Deletionen und einen pharmazeutisch annehmbaren Träger umfasst, worin die Bakterien eines Stammes mit mehreren Deletionen ein Genom aufweisen, das gentechnisch so manipuliert wurde, dass es um zumindest 5 %, um zumindest 7 %, um zumindest 14 % oder um zumindest 19 %, kleiner ist als das Genom eines nativen Ausgangsstammes
und worin es sich gegebenenfalls bei den Bakterien eines Stammes mit mehreren Deletionen um MDS42 oder MDS66 handelt.

## Revendications

1. Composition pour utilisation dans une méthode destinée à prévenir une septicémie chez un animal, comprenant une quantité efficace de bactéries *Escherichia coli* de souche de délétion multiple, où lesdites bactéries de souche de délétion multiple ont un génome qui est génétiquement modifié pour qu'il soit au moins 5%, au moins 7%, au moins 14% ou au moins 19% plus petit que le génome d'une souche parente native,
et où lesdites bactéries de souche de délétion multiple comprennent une délétion d'un ou de plusieurs gènes sélectionnés dans le groupe consistant en : b2190 ; b2191 ; b2192 ; b3215 ; b3216 ; b3217 ; b3218 ; b3219 ; b3504 ; b3505 ; b1070 ; b1071 ; b1072 ; b1073 ; b1074 ; b1075 ; b1076 ; b1077 ; b1078 ; b1079 ; b1080 ; b1081 ; b1082 ; b1083 ; b1878 ; b1879 ; b1880 ; b1881 ; b1882 ; b1883 ; b1884 ; b1885 ; b1886 ; b1887 ; b1888 ; b1889 ; b1890 ; b1891 ; b1892 ; b1893 ; b1894 ; b1917 ; b1918 ; b1919 ; b1920 ; b1921 ; b1922 ; b1923 ; b1924 ; b1925 ; b1926 ; b1927 ; b1928 ; b1929 ; b1930 ; b1931 ; b1932, b1933 ; b1934 ; b1935 ; b1936 ; b1937 ; b1938 ; b1939 ; b1940 ; b1947 ; b1942 ; b1943 ; b1944 ; b1945 ; b1946 ; b1947 ; b1948 ; b1949 ; b1950 ; b4324 ; b4325 ; b4326 ; b4327 ; b4328 ; b4329 ; b4330 ; b4331 ; b4332 ; b4333 ; b4334 ; b4335 ; b4336 ; b4337 ; b4338 ; b4339 ; b4340 ; b4341 ; b4342 ; b4345 ; b4346 ; b4347 ; b4348 ; b4349 ; b4350 ; b4351 ; b4352 ; b4353 ; b4354 ; b4355 ; b4356 ; b4357 ; b4358 ; b4484 ; b0497 ; b0498 ; b0499 ; b0500 ; b0501 ; b0502 ; b0700 ; b0701 ; b0702 ; b0703 ; b0704 ; b0705 ; b0706 ; b1356 ; b1457 ; b1458 ; b1459 ; b1460 ; b1461 ; b1462 ; b3481 ; b3482 ; b3483 ; b3484 ; b3592 ; b3593 ; b3594 ; b3595 ; b3596 ; b0981 ; b0982 ; b0983 ; b0984 ; b0985 ; b0986 ; b0987 ; b0988 ; b1021 ; b1022 ; b1023 ; b1024 ; b1025 ; b1026 ; b1027 ; b1028 ; b1029 ; b2080 ; b2081 ; b2082 ; b2083 ; b2084 ; b2085 ; b2086 ; b2087 ; b2088 ; b2089 ; b2090 ; b2091 ; b2092 ; b2093 ; b2094 ; b2095 ; b2096 ; b4438 ; b3440 ; b3441 ; b3442 ; b3443 ; b3444 ; b3445 ; bb4451 ; b3556 ; b3557 ; b3558 ; b4455 ; b1786 ; b0150 ; b0151 ; b0152 ; et b0153 de la souche MDI655 de E. coli K-12.

2. Composition pour utilisation selon la revendication 1, dans laquelle lesdites bactéries de souche de délétion multiple ont subi une délétion de celles-ci au moins des segments ADN supprimés de MDS42.

3. Composition pour utilisation selon la revendication 1, dans laquelle lesdites bactéries de souche de délétion multiple est MDS42.

4. Composition pour utilisation selon la revendication 1, dans laquelle lesdites bactéries de souche de délétion multiple est MDS66.

5. Composition pour utilisation selon l'une quelconque des revendications précédentes, dans laquelle ladite quantité efficace de bactéries de souche de délétion multiple doit être administrée avant, durant ou après un régime de traitement additionnel.

6. Composition pour utilisation selon l'une quelconque des revendications précédentes, dans laquelle lesdites bactéries de souche de délétion multiple sont des bactéries de souche de délétion multiple vivantes.

7. Composition pour utilisation selon l'une quelconque des revendications précédentes, dans laquelle ladite souche de délétion multiple *E. coli* n'a pas de gène *msbB* fonctionnel.

8. Composition pour utilisation selon l'une quelconque des revendications précédentes, dans laquelle ladite souche de délétion multiple ne possède pas un ou plusieurs gènes impliqués dans la production de lipopolysaccharide, où lesdits un ou plusieurs gènes se situent dans le groupe de gènes rfa entre le nucléotide 3792010 et 3806121 du chromosome *E*. *coli ;* et
où en option, lesdits un ou plusieurs gènes sont sélectionnés dans le groupe consistant en *rfal, rfaJ* et rfe ou combinaisons de ceux-ci.

9. Composition pour utilisation selon l'une quelconque des revendications précédentes, dans laquelle ladite souche de délétion multiple ne possède pas un ou plusieurs gènes impliqués dans la production d'un antigène commun entérique, où lesdits un ou plusieurs gènes se situent sur le chromosome E. coli entre le nucléotide 3965939 et 3980295.

10. Composition pour utilisation selon l'une quelconque des revendications précédentes, dans laquelle dans ladite méthode :
(a) ladite composition doit être administrée à l'animal en une seule dose ;
(b) des doses multiples de ladite composition doivent être administrées à l'animal ; ou
(c) des doses multiples de ladite composition doivent être administrées à l'animal, et l'administration de ladite composition est hebdomadaire.

11. Composition pour utilisation selon l'une quelconque des revendications précédentes, dans laquelle dans ladite méthode, ledit animal est un humain.

12. Composition pour utilisation selon l'une quelconque des revendications précédentes, dans laquelle lesdites bactéries de souche de délétion multiple ont un génome qui est génétiquement modifié pour être au moins 5% et jusqu'à 20% plus petit que le génome d'une souche parente native.

13. Composition pour utilisation selon l'une quelconque des revendications précédentes, dans laquelle ladite méthode est une méthode pour prévenir une septicémie provoquée par l'exposition à des bactéries gram-négatives.

14. Composition pour utilisation selon la revendication 13, dans laquelle dans ladite méthode, la bactérie gram-négative est *E*. *coli.*

15. Composition pharmaceutique comprenant des bactéries de souche de délétion multiple et un support acceptable du point de vue pharmaceutique, où lesdites bactéries de souche de délétion multiple ont un génome qui est génétiquement modifié pour qu'il soit au moins 5%, au 7%, au moins 14% ou au moins 19% plus petit que le génome de sa souche parente native ;
et où en option, ladite bactérie de souche de délétion multiple est MDS42 ou MDS66.
